# EUROPEAN PATENT APPLICATION

(11) **EP 1 959 006 A1**
(43) Date of publication of application: **20.08.2008**
(21) Application number: 07253300.3
(22) Date of filing: 21.08.2007
(51) Int. Cl.: C12N 1/20

(54) **Mutant strain of Amycolatopsis orientalis and process for preparing vancomycin hydrochloride**

(30) Priority: 14.02.2007 KR 20070015271
(71) Applicant: Biongene Co. Ltd., Chongro-ku Seoul (KR)
(72) Inventor: Kim, Sang Yong, Gwangmyeong-Shi, Gyeonggi-Do (KR); Kim, Do Sun, Yongsan-Ku, Seoul (KR); Jung, Hyung Moo, Eunpyeong-Ku, Seoul (KR); Lee, Jung Kul, Paldal-Ku, Suwon-Shi, Gyeonggi-Do (KR)
(74) Representative: Harding, Charles Thomas

(57) **Abstract**

The present invention provides a process for preparing vancomycin hydrochloride using a mutant strain of *Amycolatopsis orientalis* (accession No. KCCM-10836P) comprising the steps of i) mutating and isolating a strain of *Amycolatopsis orientalis* (accession No. KCCM-10836P) for producing vancomycin from mother strain of *Amycolatopsis orientalis* (accession No. ATCC-19795) using NTG(N-methyl-N'-nitro-N-nitrosoguanidine) in the selection medium; ii) seed culturing the isolated mutant strain of *Amycolatopsis orientalis* (accession No. KCCM-10836P); iii) cultivating and fermenting said mutant strain of *Amycolatopsis orientalis* in the fermentation medium consisting of 12~18(w/v)% of dextrin, 2.2~3.8(w/v)% of bean powder, 1.9~2.9(w/v)% of potato protein, 0.10~0.14(w/v)% of sodium chloride and a small amount of minerals; iv) filtering vancomycin using microfilter in the cultivation broth by removing mycelia; v) purifying obtained vancomycin using column system compacted with cation exchange resin, anion exchange resin and absorbent resin; and vi) crystallizing the purified vancomycin hydrochloride with ammonium chloride and ethanol.

## Description

### BACKGROUND OF THE INVENTION

### Field of the Invention

This invention relates to a mutant strain of *Amycolatopsis orientalis* (accession No. KCCM-10836P) for producing vancomycin, a fermentation process, and a purification process for preparing vancomycin hydrochloride using this mutant strain. More particularly, the present invention relates to i) a fermentation process for preparing vancomycin using this mutant strain in a high yield and productivity in the medium containing dextrin as carbon source, soybean powder or potato protein as nitrogen source and other minerals by controlling the fermentation conditions, and ii) a purification method composed of a column system and a process for crystallizing vancomycin hydrochloride.

### Description of Prior Art

Vancomycin is one of glycopeptide antibiotics inhibiting the synthesis of cell walls of microorganism. Further, vancomycin exhibits a strong inhibition effect on gram positive bacteria, such as, *Streptococci, Staphylococci* and *Clostridium difficile,* which are gram positive bacteria resistant to penicillin and cephalosporin antibiotics. Also, vancomycin has been known to have high treating effects on the diseases derived from methicillin-resistant *Staphylococcus aureus* (MRSA), which is fatal to post-operation patients, elderly patients and patients having weak immunity.

In U.S. Pat. No. 3, 067, 099, the process for fermenting and producing vancomycin has been disclosed. However, this patented process showed a very low strain yield and productivity of 0.2 mg/mL. Further, the production cost had been also very high.

To solve above problems, lots of strains having a high strain yield of vancomycin have been isolated and developed. In Korean laying-open Patent Publication No.93-13090, mutated strain of *Amycolatopsis* (accession No.KFCC-10745) has been disclosed to produce vancomycin in a high strain yield. On the other hand, in Korean laying-open Patent Publication No. 93-13091, mutated strain of *Amycolatopsis orientalis* (accession No. KFCC-10744) has been disclosed to produce vancomycin in a high strain yield. Further, in Korean laying-open Patent Publication No. 93-13090, a fermentation process for producing vancomycin in a high yield using the cultivation medium containing 1.0~2.0 w/v% of gluten or soybean cake as nitrogen source has been disclosed.

Even though said *Amycolatopsis* strains disclosed in above patents showed a high strain yield of 6.1mg/mL for producing vancomycin, there has been lots of handicaps for commercializing vancomycin, such as, high toxicity and high impurity contents as well as high cost for production.

To solve above problems the present invention has isolated and developed a novel mutant strain of *Amycolatopsis* with characteristics of very high vancomycin productivity and low impurity contents as well as fermentation process for producing vancomycin using this isolated mutant strain in the medium containing dextrin as carbon source, bean powder or potato protein as nitrogen source and other trace elements by adjusting the medium composition and fermentation conditions. Further, the present invention also affords an efficient process purifying vancomycin from fermentation broth assuring high purity, low impurity, and low toxicity.

### SUMMARY OF THE INVENTION

The object of the present invention is to provide a mutant strain of *Amycolatopsis orientalis* (accession No. KCCM-10836P) for producing vancomycin.

Another object of the present invention is to provide a process for preparing vancomycin hydrochloride using a mutant strain of *Amycolatopsis orientalis* (accession No. KCCM-10836P) comprising the steps of i) mutating and isolating a strain of *Amycolatopsis orientalis* (accession No. KCCM-10836P) for producing vancomycin from mother strain of *Amycolatopsis orientalis* (accession No. ATCC-19795) using NTG(N-methyl-N'-nitro-N-nitrosoguanidine) in the selection medium; ii) seed culturing the isolated mutant strain of *Amycolatopsis orientalis* (accession No. KCCM-10836P); iii) cultivating and fermenting said mutant strain of *Amycolatopsis orientalis* in the fermentation medium consisting of 12~18(w/v)% of dextrin, 2.2~3.8(w/v)% of bean powder, 1.9~2.9(w/v)% of potato protein, 0.10~0.14(w/v)% of sodium chloride and a small amount of minerals; iv) filtering vancomycin using microfilter in the cultivation broth by removing mycelia; v) purifying obtained vancomycin using column system compacted with cation exchange resin, anion exchange resin and absorbent resin; and vi) crystallizing the purified vancomycin hydrochloride with ammonium chloride and ethanol.

Further, said fermentation medium further comprises 0.10~0.14(w/v)% of sodium chloride, 1~100 mg/L of calcium, 1~100mg/L of pyridoxine, 1~100mg/L of melanin biosynthesis inhibitors selected from arbutin, hydroquinone and tricyclazole.

Further, said purifying step using column system comprises the steps of i) passing the broth through adsorption column compacted with hydrophobic absorbent resin, wherein vancomycin remains in the column and impurities are passed through, and vancomycin is eluted by 5~15% of ethanol; ii) passing the eluent obtained in step (i) through a column compacted with strong acid cation exchange resin, wherein a solution comprising vancomycin is passed through and impurities remain in the column; iii) passing the solution obtained in step (ii) through a column compacted with weak base anion exchange resin, wherein a solution comprising vancomycin is passed through and impurities remain in the column; iv) treating the solution obtained in step (iii) with activated carbon to remove colorants and impurities; v) treating decolorized solution with activated alumina resin, and vancomycin is eluted with 35~60% of isopropanol; and vi) crystallizing vancomycin hydrochloride.

### BRIEF DESCRIPTION OF THE DRAWINGS

FIG. 1 is a view illustrating an HPLC analytical result of the cultivation mixture of the mutant strain using a method according to the embodiment of the present invention. Main peak at 6.466 minutes shows vancomycin peak. The vancomycin peak area indicates that the contents of vancomycin shall be 76.447% of total vancomycin cultivation mixture.
FIG.2 is a view illustrating an HPLC analytical result of the cultivation mixture of the mother strain. Main peak at 6.489 minutes shows vancomycin peak. The vancomycin peak area indicates that the contents of vancomycin shall be 52.080% of total vancomycin cultivation mixture.
FIG. 3 is a view illustrating an HPLC analytical result of vancomycin hydrochloride purified using the method in Example 9 of the present invention.
FIG. 4 is a view illustrating an HPLC analytical result of standard vancomycin cited in U.S. Pharmacopoeia (Cat. No. USP 1709007).

### DETAILED DESCRIPTION OF THE INVENTION

The mutant strain of *Amycolatopsis orientalis* (accession No. KCCM-10836P) has been isolated by following 3 steps of processes.

The first step of isolation of mutant strain is to select the strain having high vancomycin resistance. *Amycolatopsis orientalis* (accession No. ATCC-19795) is used as mother strain. After mutating the mother strain using NTG (N-methyl-N'-nitro-N-nitrosoguanidine), the mutated strains have been cultured in the selection medium containing 0.1~50g/L of vancomycin for 4 days at 28~32°C. The mutated strains showing high growth in the selection medium have been selected and isolated. The other composition of selection medium is shown at Table 1.

**[Table 1] Composition of selection medium**

| Component | Concentration |
|---|---|
| Soluble starch | 1.0% |
| Bacto soytone | 1.0% |
| Agar | 2% |

The second step of isolation of mutant strain is to select the strain having high vancomycin productivity from the mutant strains selected in the first step. After the mutated strains have been cultured on the surface of agar plate with the selection medium without vancomycin for 4 days, *Bacillus subtilis* ATCC-6633 culture was overlayed on the surface of the agar plate, and then it was incubated for 24 hours at 30°C. The mutated strains showing high growth inhibitory effect on *Bacillus subtilis* have been selected and isolated. The other composition of medium is same as shown in Table 1.

The third step of isolation of mutant strain is to treat the obtained mutant strain with 250-280 nm of UV. Then, the mutant strain with characteristics of both high vancomycin productivity and low impurity contents has been selected and isolated as a final strain.

The isolated mutant strain of *Amycolatopsis orientalis* was deposited with accession No. KCCM-10836P at Korea Culture Center of Microorganisms (KCCM) located at 361-221, Yurim B/D Hongje-1-dong, Seodaemun-gu, Seoul 120-091, Republic of Korea on January 12, 2007 under Budapest Treaty. The major characteristics of the mutant strain (accession No. KCCM-10836P) are high vancomycin productivity, high resistance to vancomycin itself, and low impurity contents. Therefore, the culture of this mutant strain shows high productivity of vancomycin, good growth in the medium containing vancomycin, and low productivity of impurity, specially, appearing just before the vancomycin peak on the HPLC chromatogram.

The cultivation and analytical method of isolated mutant strain can be described as follows.

The mutant strain has been cultured in the medium at 23~37°C, pH 5.5~8.0 and 200-700 rpm using 50ml Erlenmeyer flask, Petri-dish, or 7L jar fermenter. The concentration of vancomycin has been determined by the HPLC analysis based on *US Pharmacopeia* 28. The growth of microorganism has been measured by PMV (packed mycelia volume) after centrifuging the 10 mL of culture broth at 450 g for 10 minutes. The concentration of dissolved oxygen has been measured using electrode made by Ingold Co.(Swiss, polarographic type).

Table 2 shows the vancomycin resistance among mother strain (ATCC-19795), *Amycolatopsis orientalis* (KFCC-10990) and *Amycolatopsis orientalis* (KCCM-10836P).

**[Table 2]**

| Strain | Vancomycin (g/L) | | | | | | | | | |
|---|---|---|---|---|---|---|---|---|---|---|
| | 0 | 0.5 | 1.0 | 5.0 | 10.0 | 15.0 | 20.0 | 25.0 | 30.0 | 35.0 |
| Mother strain (ATCC-19795) | +++ | +++ | ++ | + | - | - | - | - | - | - |
| Mutant strain (KFCC-10990) | +++ | +++ | + | + | + | - | - | - | - | - |
| Mutant strain (KCCM-10836P) | +++ | +++ | +++ | +++ | +++ | ++ | + | - | - | - |

Further, Table 3 shows the morphological, physiological and biochemical characteristics among mother strain (ATCC-19795), *Amycolatopsis orientalis* (KFCC-10990) and *Amycolatopsis orientalis* (KCCM-10836P).

**[Table 3]**

| Characteristic | Mother strain (ATCC-19795) | Mutant strain (KFCC-10990) | Mutant strain of present invention (KCCM-10836P) |
|---|---|---|---|
| Type of filament | branched | branched | branched |
| Color of colony | yellow | white | white |
| Spore formation | + | + | ++ |
| Gram staining | + | + | + |
| Oxygen-demanding | aerobic | aerobic | aerobic |
| Nutrient-demanding | - | - | - |
| Movement | - | - | - |
| Optimal Temperature | 25-37°C | 28-38°C | 23-35°C |
| Optimal pH | 6.5-8.0 | 6.8-8.0 | 5.5-7.5 |
| Use of glucose | + | + | + |
| Use of fructose | + | + | + |
| Use of maltose | + | + | + |
| Use of raffinose | - | - | - |

The isolated mutant strain (accession No. KCCM-10836P) and the mother strain have been cultured in 7 L jar fermenter with 4 L of production medium at 34°C, 700rpm for 120 hours. After removing mycelia, the amount of vancomycin, growth (PMV), the purity of vancomycin, and the contents of impurity have been determined by HPLC analysis based on *US Pharmacopeia* 28*.*

Table 4 shows the composition of production medium.

**[Table 4]**

| Component | Concentration |
|---|---|
| Dextrin | 15 % |
| Soybean flour | 1.8% |
| Potato protein | 1.8% |
| NaCl | 0.12% |

Table 5 shows growth (PMV), vancomycin productivity, purity of vancomycin, and impurity contents of the cultivation mixture of mother strain and mutant strain of present invention.

**[Table 5]**

| Strain | Growth(PMV) | Vancomycin(g/L) | Purity of Vancomycin (%area) | Major Impurity (%area)¹ |
|---|---|---|---|---|
| Mother strain (ATCC-19795) | 28 | 3.3 | 52.08 | 22.94 |
| Mutant strain of present invention (KCCM-10836P) | 40 | 7.1 | 76.47 | 0.35 |

| | | | | |
|---|---|---|---|---|
| ¹ : Major impurity means the peak at 5.95min of retention time just before the peak of vancomycin on the HPLC chromatogram | | | | |

As shown in Table2 and Table 3, the mutant strain of present invention has characteristics different from those of other strains in respect to the resistance to vancomycin itself, the optimal cultivation temperature, and the optimal pH.

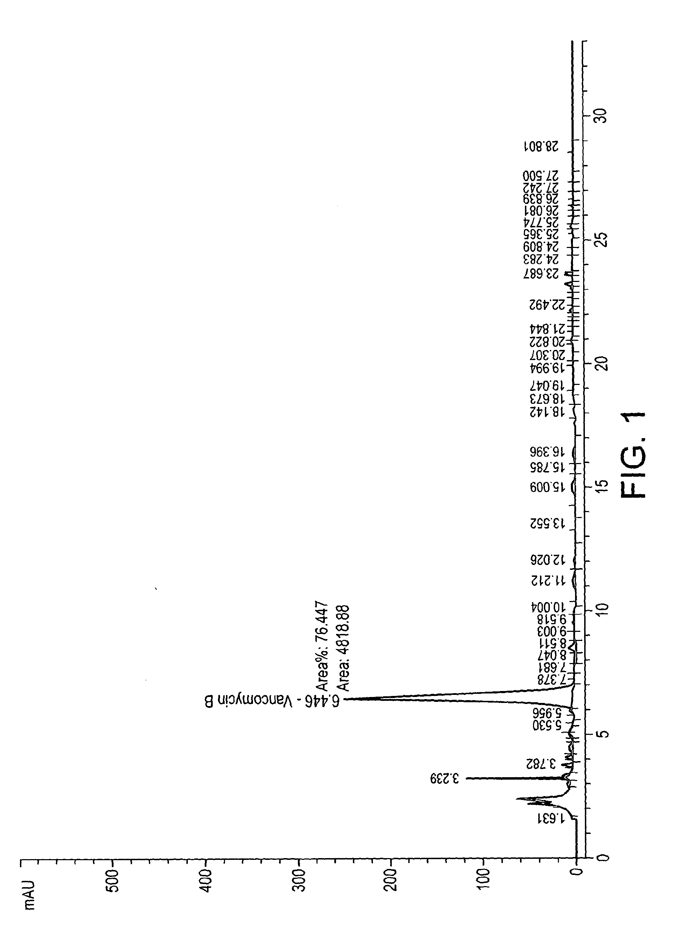

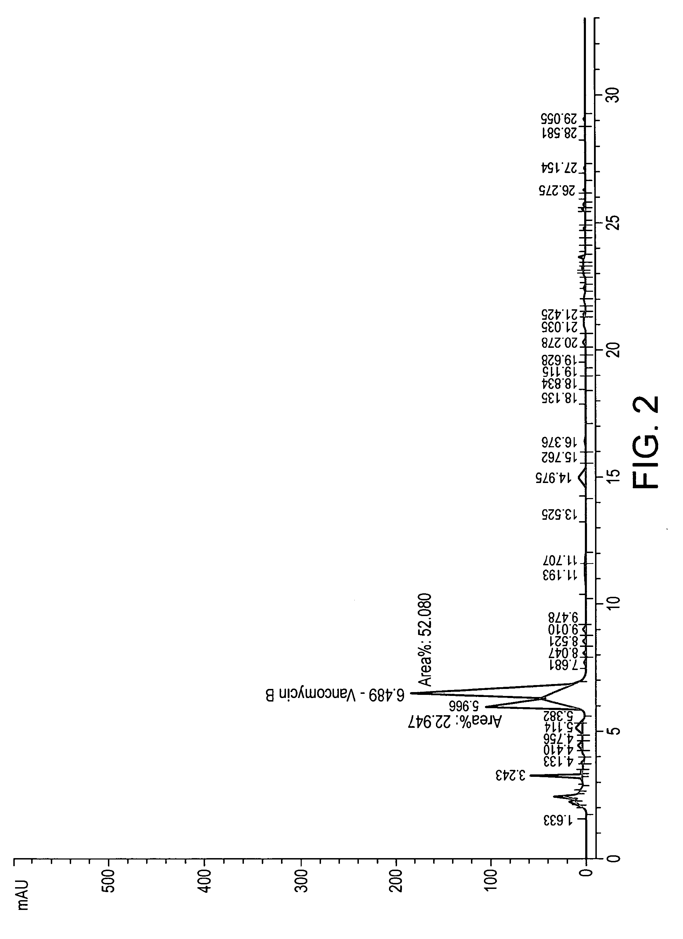

Furthermore, the mutant strain produce vancomycin with much higher productivity and make the major impurity (5.95mim) much less than the mother strain as shown in Table5, FIG.1, and FIG.2.

The present invention can be explained by following examples. However, the scope of present invention shall not be limited by following examples

### EXAMPLES

### (Example 1) Vancomycin production in a 7L-jar fermenter

The production of vancomycin by mutant strain of *Amycolatopsis orientalis* (KCCM-10836P) has been measured in a 7L-jar fermenter.

### (Seed culture)

The isolated mutant strain has been seed-cultured in a 500mL of Erlenmeyer flask containing 50mL of 1^{st} seed medium at 30°C and 250 rpm for 24 hours. Then, 0.1mL of seed culture has been transferred to a 500mL of Erlenmeyer flask containing 100mL of 2^{nd} seed medium and incubated at 30°C and 250 rpm for 60 hours.

Table 6 shows the composition of 1^{st} seed medium

**[Table 6]**

| Component | Concentration |
|---|---|
| Glucose | 1.7% |
| Malt extract | 0.3% |
| Yeast extract | 0.3% |
| Peptone | 1.1 % |

Table 7 shows the composition of 2^{nd} seed medium

**[Table 7]**

| Component | Concentration |
|---|---|
| Dextrin | 5.0% |
| Soybean flour | 0.5% |
| Potato protein | 0.5% |
| CaCO₃ | 0.3% |

### (Main culture)

The main culture has been carried out in a 7L-jar fermenter chareged with 4L of production medium (Table 4) containing 5-20 % of dextrin. 2^{nd} seed culture (200ml) has been transferred to a 7L-jar fermenter. Temperature, aeration rate, and pH during the cultivation have been maintained at 34°C, 1 vvm, and 7.5, respectively. Dissolved oxygen concentration has been maintained more than 20% with stirring in the range of 700-900 rpm. To evaluate the effect of the initial concentrations of dextrin on vancomycin production, various concentrations of dextrin (5~20%) have been used. Vancomycin production was the highest at 15% of dextrin concentration; therefore, this value represents the optimal concentration of dextrin for vancomycin production.

Table 8 shows the productivity of vancomycin according to the variation of dextrin concentration.

**[Table 8]**

| Dextrin(%) | Vancomycin(g/L) | Productivity(g/L · h) |
|---|---|---|
| 5 | 6.1 | 0.050 |
| 10 | 7.2 | 0.060 |
| 15 | 7.5 | 0.062 |
| 20 | 7.0 | 0.058 |

### (Example 2) Vancomycin production according to the variation of pH

The vancomycin production has been measured according to the variation of pH. The same fermentation medium with 15% of dextrin and method in Example 1 has been employed. Only pH was varied from 5.5 to 8.0 Table 10 shows the productivity of vancomycin according to the variation of pH.

**[Table 9]**

| pH | Vancomycin(g/L) | Productivity(g/L · h) |
|---|---|---|
| 5.5 | 6.0 | 0.050 |
| 6.5 | 7.2 | 0.060 |
| 7.5 | 7.6 | 0.063 |
| 8.0 | 4.0 | 0.033 |

### (Example 3) Vancomycin production according to the variation of temperature

The vancomycin production has been measured according to the variation of temperature. The same fermentation medium and method in Example 2 has been employed. The pH has been adjusted to 7.5. Temperature was varied from 23°C to 37°C

Table 10 shows the productivity of vancomycin according to the variation of temperature.

**[Table 10]**

| Temperature | Vancomycin(g/L) | Productivity(g/L · h) |
|---|---|---|
| 23 | 5.5 | 0.045 |
| 28 | 7.0 | 0.058 |
| 34 | 7.5 | 0.062 |
| 37 | 4.4 | 0.036 |

### (Example 4) Vancomycin production according to the variation of concentration of a nitrogen source (soybean flour)

The vancomycin production has been measured according to the variation of concentration of nitrogen source (soybean flour). The same fermentation medium and method in Example 3 except soybean flour and temperature has been employed. Temperature has been adjusted to 32°C and soybean flour has been varied from 1.0% to 3.5%. The medium containing 3% of soybean flour shows the optimal.

Table 11 shows the productivity of vancomycin according to the variation of concentration of soybean flour.

**[Table 11]**

| Nitrogen source (Soybean flour) (%) | Vancomycin(g/L) | Amount of strain (PMV)(%) | Productivity(g/L · h) |
|---|---|---|---|
| 1.0 | 7.0 | 38 | 0.058 |
| 1.8 | 7.5 | 42 | 0.062 |
| 2.4 | 9.0 | 46 | 0.075 |
| 3.0 | 9.2 | 45 | 0.076 |
| 3.5 | 8.9 | 41 | 0.074 |

### (Example 5) Vancomycin production according to the variation of concentration of a nitrogen source (potato protein)

The vancomycin production has been measured according to the variation of concentration of a nitrogen source (potato protein). The fermentation medium comprising dextrin (15%), soybean flour (3.0%), potato protein (1.0-3.5%) and NaCl (0.12%) and method in Example 4 has been employed. The medium containing 2.4% of potato protein shows the optimal.

Table 12 shows the productivity of vancomycin according to the variation of concentration of potato protein.

**[Table 12]**

| Nitrogen source (potato protein)(%) | Vancomycin(g/L) | Amount of strain (PMV)(%) | Productivity(g/L · h) |
|---|---|---|---|
| 1.0 | 8.0 | 43 | 0.066 |
| 1.8 | 9.0 | 46 | 0.075 |
| 2.4 | 9.5 | 46 | 0.079 |
| 3.0 | 9.2 | 42 | 0.076 |
| 3.5 | 7.5 | 38 | 0.062 |

### (Example 6) Vancomycin production according to the variation of concentration of CaCl_{2 •} 2H₂O

The vancomycin production has been measured according to the variation of concentration of CaCl₂ 2H₂O. The fermentation medium comprising dextrin (15%), soybean flour (3%), potato protein (2.4%), and NaCl (0.12%) and the same method in Example 5 has been employed. The 40mg/L of CaCl₂ 2H₂O showed the optimal.

Table 13 shows the productivity of vancomycin according to the variation of concentration of CaCl₂ 2H₂O.

**[Table 13]**

| CaCl₂ · 2H₂O (mg/L) | Vancomycin(g/L) | Amount of strain (PMV)(%) | Productivity(g/L · h) |
|---|---|---|---|
| 0 | 9.5 | 46 | 0.079 |
| 10 | 9.8 | 48 | 0.081 |
| 20 | 10.3 | 50 | 0.085 |
| 40 | 10.9 | 55 | 0.090 |
| 80 | 8.9 | 42 | 0.074 |
| 100 | 8.5 | 42 | 0.070 |

### (Example 7) Vancomycin production according to the variation of pyridoxine

The vancomycin production has been measured according to the variation of pyridoxine concentration. The same fermentation medium supplemented with 40mg/L of CaCl₂ · 2H₂O and method in Example 6 has been employed. The 50mg/L of pyridoxine showed the optimal.

Table 14 shows the productivity of vancomycin according to the variation of pyridoxine concentration.

**[Table 14]**

| Pyridoxine(mg/L) | Vancomycin(g/L) | Amount of strain (PMV)(%) | Productivity(g/L · h) |
|---|---|---|---|
| 0 | 10.9 | 46 | 0.090 |
| 10 | 10.8 | 46 | 0.091 |
| 20 | 11.2 | 50 | 0.093 |
| 40 | 11.5 | 52 | 0.095 |
| 50 | 12.1 | 56 | 0.100 |
| 80 | 10.6 | 50 | 0.088 |
| 100 | 10.6 | 51 | 0.088 |

### (Example 8) Vancomycin production according to the addition of melanin biosynthesis inhibitor

The vancomycin production has been measured according to addition of melanin inhibitor. The same fermentation medium supplemented with 40mg/L of CaCl₂ · 2H₂O and 50mg/L of pyridoxine and method in Example 6 has been employed. The melanin biosynthesis inhibitor has been selected from arbutin, hydroquinone and tricyclazole. The addition of 50mg/L of melanin biosynthesis inhibitor shows better productivity compared to that of control without melanin biosynthesis inhibitors

Table 15 shows the productivity of vancomycin according to the addition of melanin biosynthesis inhibitors.

**[Table 15]**

| Melanin Biosynthesis inhibitor (50 mg/L) | Vancomycin(g/L) | Amount of strain (PMV)(%) | Productivity(g/L· h) |
|---|---|---|---|
| 0 | 12.1 | 43 | 0.100 |
| Arbutin | 12.3 | 46 | 0.102 |
| Hydroquinone | 13.5 | 46 | 0.112 |
| Tricyclazole | 12.6 | 42 | 0.105 |

### (Example 9) Purification of vancomycin

200 L of a fermentation broth obtained from 300L fermenter containing vancomycin in a concentration of 14 g/L was mixed with 200L of distilled water and pH was adjusted to 2-3 by adding 2N hydrochloric acid. 400L of diluted fermentation broth was filtered using micro-filtration system. As soon as the filtrate was obtained, 2N sodium hydroxide was added to adjust pH to 7-8.

The filtrate was passed through columns comprising 40L of hydrophobic absorbent resin (HP20, Diaion, Japan). Then, vancomycin absorbed to hydrophobic absorbent resin was eluted with 5-15% ethanol at the flow rate of 40L per hour. Consequently, the eluent containing vancomycin is passing through 20L of strong cation exchange resin (SK1B, Diaion, Japan) and 20L of weak anion exchange resin (WA30, Diaion, Japan) continuously at a flow rate of 60 L per hour. The columns were washed with 200L of distilled water as same sequence as the loading of filtrate. The fractions containing vancomycin were combined, homogenized, and concentrated to 100 g/L using nano-filtration system

The concentrate (14L) was mixed with equal volume of isoprophyl alcohol and then 2N sodium hydroxide was added to adjust pH to 4-5. Activated carbon (840g) was added to the mixture, agitated slowly for 1 hour and then filtered off with a filter of diatomaceous earth filter.

The filtrate was passed through a column packed with 9L of activated alumina (Wako, Japan) which is conditioned to acidic form at a flow rate of 9L per hour. After the loading of filtrate was completed, 50% isoprophyl alcohol was flushed through the column at the same flow rate. The solution from outlet of the column was fractioned and analyzed by HPLC to check the purity of vancomycin. The fractions with not less than 93% of chromatographic purity of vancomycin were combined, homogenized and concentrated up to 150 g/L by nano-filtration system.

The concentrate containing at least 150 g/L of vancomycin with more than 93% of chromatographic purity was adjusted to pH 2-3 with 2N hydrochloric acid, and then the conductivity of the concentrate was increased to 15-20 ms/cm by adding aqueous solution saturated with ammonium chloride of pharmaceutical grade. Next, the concentrate was passed through a filter with 0.2 µm pore to remove endotoxin as well as to assure sterility, and then it was left at 20-30°C for 12 hours.

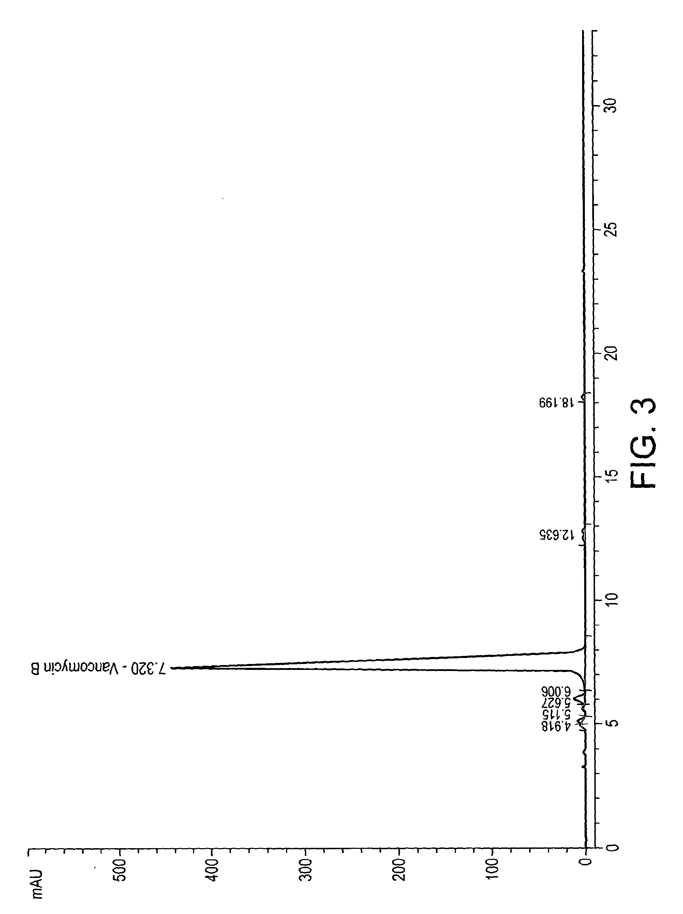

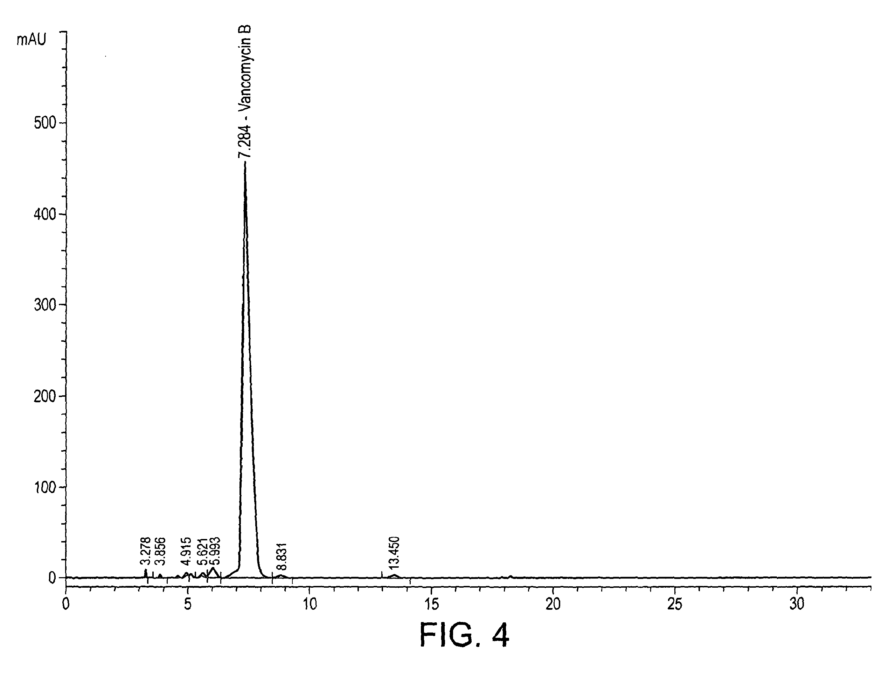

Next, chilled absolute ethanol was added to the concentrate drop by drop until vancomycin hydrochloride was crystallized, the slurry was left in refrigerator for 12 hours to accelerate the crystallization, and then the slurry was filtered off. The filtered precipitate was dried in a vacuum dryer at 40°C. The dried vancomycin hydrochloride had a weight of 840g and was white and freely soluble powder with at least 95% chromatographic purity and 1050 U of antimicrobial activity by analysis based on *U.S. Pharmacopeia* 28. The analytical results are illustrated in FIG.3 and FIG.4.

## Claims

1. A mutant strain of *Amycolatopsis orientalis* (accession No. KCCM-10836P) for producing vancomycin.

2. A process for preparing vancomycin hydrochloride using a mutant strain of *Amycolatopsis orientalis* (accession No. KCCM-10836P) comprising the steps of
i) mutating and isolating a strain of *Amycolatopsis orientalis* (accession No. KCCM-10836P) for producing vancomycin from mother strain of *Amycolatopsis orientalis* (accession No. ATCC-19795) using NTG(N-methyl-N'-nitro-N-nitrosoguanidine) in the selection medium;
ii) seed culturing the isolated mutant strain of *Amycolatopsis orientalis* (accession No. KCCM-10836P);
iii) cultivating and fermenting said mutant strain of *Amycolatopsis orientalis* in the fermentation medium consisting of 12~18(w/v)% of dextrin, 2.2~3.8(w/v)% of bean powder, 1.9~2.9(w/v)% of potato protein, 0.10~0.14(w/v)% of sodium chloride and a small amount of minerals;
iv) filtering vancomycin using microfilter in the cultivation broth by removing mycelia;
v) purifying obtained vancomycin using column system compacted with cation exchange resin, anion exchange resin and absorbent resin; and
vi) crystallizing the purified vancomycin hydrochloride with ammonium chloride and ethanol.

3. The process for preparing vancomycin hydrochloride according to claim 2, wherein said fermentation medium further comprises 0.10~0.14(w/v)% of sodium chloride, 1-100 mg/L of calcium, 1~100mg/L of pyridoxine, 1~100mg/L of melanin biosynthesis inhibitors selected from arbutin, hydroquinone and tricyclazole.

4. The process for preparing vancomycin hydrochloride according to any one of claims 2 and 3, wherein said purifying step using column system comprises the steps of
i) passing the broth through adsorption column compacted with hydrophobic absorbent resin, wherein vancomycin remains in the column and impurities are passed through, and vancomycin is eluted by 5-15% of ethanol;
ii) passing the eluent obtained in step (i) through a column compacted with strong acid cation exchange resin, wherein a solution comprising vancomycin is passed through and impurities remain in the column;
iii) passing the solution obtained in step (ii) through a column compacted with weak base anion exchange resin, wherein a solution comprising vancomycin is passed through and impurities remain in the column;
iv) treating the solution obtained in step (iii) with activated carbon to remove colorants and impurities;
v) treating decolorized solution with activated alumina resin, and vancomycin is eluted with 35~60% of isopropanol; and
vi) crystallizing vancomycin hydrochloride.

5. A process for preparing vancomycin comprising cultivating and fermenting a mutant strain of *Amycolatopsis orientalis* (accession No. KCCM-10836P) in a fermentation medium.

6. A process according to claim 5 wherein the vancomycin is purified.

7. A process according to claim 6 wherein the vancomycin is crystallized.

8. A process according to any one of claims 5 to 7 wherein the fermentation medium comprises dextrin, bean powder, potato protein, sodium chloride and minerals.

9. A process according to claim 8, wherein said fermentation medium further comprises sodium chloride, calcium, pyridoxine, and melanin biosynthesis inhibitors.

10. A process according to any one of claims 5 to 9 wherein the vancomycin is vancomycin hydrochloride.
